(19) Europäisches Patentamt
European Patent Office
Office européen des brevets

(11) Publication number: **0 125 315**
**A1**

(12) # EUROPEAN PATENT APPLICATION

published in accordance with Art. 158(3) EPC

(21) Application number: **83903564.9**

(22) Date of filing: **09.11.83**

Data of the international application taken as a basis:

(86) International application number:
**PCT/JP83/00398**

(87) International publication number:
**WO84/01945 (24.05.84 84/13)**

(51) Int. Cl.[3]: **C 07 D 213/64**
C 07 D 213/65, C 07 D 213/68
C 07 D 239/34, C 07 D 277/34
A 61 K 31/425, A 61 K 31/44
A 61 K 31/445, A 61 K 31/505

(30) Priority: **09.11.82 JP 197278/82**

(43) Date of publication of application:
**21.11.84 Bulletin 84/47**

(84) Designated Contracting States:
**BE DE FR GB SE**

(71) Applicant: **Yoshitomi Pharmaceutical Industries, Ltd.**
**35 Hiranomachi 3-chome Higashi-ku**
**Osaka-shi Osaka 541(JP)**

(72) Inventor: **MURO, Tomio**
**631-7 Shiohama Kakizemachi Nakatsu-shi**
**Oita 871(JP)**

(72) Inventor: **TSUDA, Yoshinao**
**2274 Kanaya-Uenocho Nakatsu-shi**
**Oita 871(JP)**

(72) Inventor: **MISHINA, Tadashi**
**925-6 Oaza-Hirotsu Yoshitomimachi**
**Chikujogun Fukuoka 871(JP)**

(72) Inventor: **OBATA, Minoru**
**4-36 Okidaimachi Nakatsu-shi**
**Oita 871(JP)**

(72) Inventor: **INUI, Jun**
**11-916, No. 6, Toshima 5-chome Kita-ku**
**Tokyo 114(JP)**

(74) Representative: **von Kreisler, Alek, Dipl.-Chem. et al,**
**Deichmannhaus am Hauptbahnhof**
**D-5000 Köln 1(DE)**

(54) **PHENOXYAMINOPROPANOL DERIVATIVES.**

(57) Phenoxyaminopropanol derivatives represented by the general formula (I), wherein R represents 2-, 3- or 4-pyridyl, 2-pyrimidyl, 2-thiazolyl or 5-bromo-2-thiazolyl, $R^1$ and $R^2$ each represents H, lower alkyl, cycloalkyl, aralkyl or, when bound to each other, $R^1$ and $R^2$ represent a group forming a pyrrolidine or piperidine ring together with the adjacent nitrogen atom, and A represents alkylen containing 2 or 3 carbon atoms, and salts thereof. These are useful as heart-selective β-blockers.

**EP 0 125 315 A1**

0125315



# S P E C I F I C A T I O N

## PHENOXYAMINOPROPANOL DERIVATIVES

### Technical Field and Disclosure of The Invention

This invention relates to novel and therapeutically useful phenoxyaminopropanol derivatives represented by the generic formula:

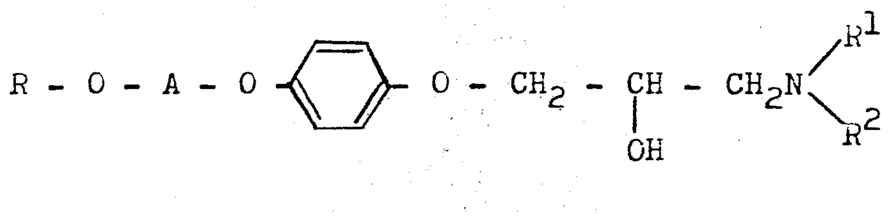 (I)

and salts thereof.

In the above formula, R represents 2-, 3- or 4-pyridyl, 2-pyrimidyl, 2-thiazolyl or 5-bromo-2-thiazolyl; $R^1$ and $R^2$ each represent hydrogen, lower alkyl (methyl, ethyl, propyl, isopropyl, butyl, isobutyl, tert-butyl, etc.), cycloalkyl (cyclohexyl, etc.) or aralkyl (benzyl, phenethyl, 3,4-dimethoxyphenethyl, 3-phenylpropyl, etc.), or form, together with the adjacent nitrogen atom, pyrrolidine or piperidine; and A represents alkylene containing 2 or 3 carbon atoms.

The objective compounds of formula (I) can be produced by reacting a compound of the general formula:

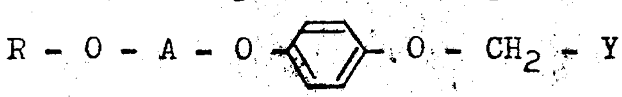 (II)

(wherein Y represents 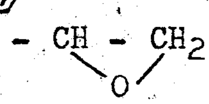 or $- CH(OH)- CH_2 -$ Hal

(Hal is a halogen atom), and A and R are as defined above) with an amine of the general formula:

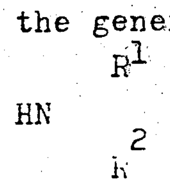 (III)

(wherein $R^1$ and $R^2$ are as defined above).

The reaction is preferably carried out in the presence of a solvent such as diethyl ether, dioxane, tetrahydrofuran, methanol, ethanol, propanol, isopropanol, benzene, toluene, xylene, water, dimethylformamide, dimethylsulfoxide, acetonitrile, or such other solvent that doesn't inhibit the reaction.

Where a compound of formula (II) wherein Y is $-CH(OH)-CH_2-Hal$ and an amine of formula (III) are reacted, an excess of the amine compound may be used as a deacidifying agent or otherwise an alkali carbonate such as potassium carbonate, sodium carbonate, etc. or a tertiary amine such as triethylamine, pyridine, etc. may be used as a deacidifying agent.

Alternatively, the objective compounds of formula (I) can be produced by reacting a phenolic derivative of the general formula:

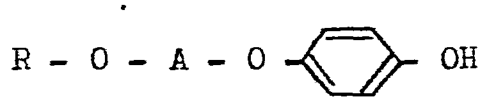

(IV)

(wherein A and R are as defined above) with an oxazolidine derivative of the general formula:

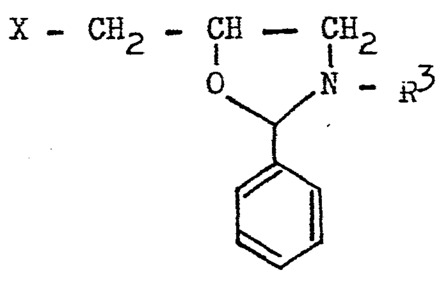

(V)

(wherein X represents a reactive derivative of alcohol,

e.g. methylsulfonyloxy, p-tolylsulfonyloxy or a halogen atom; and $R^3$ represents alkyl or aralkyl)
and subjecting the resulting compound of the general formula:

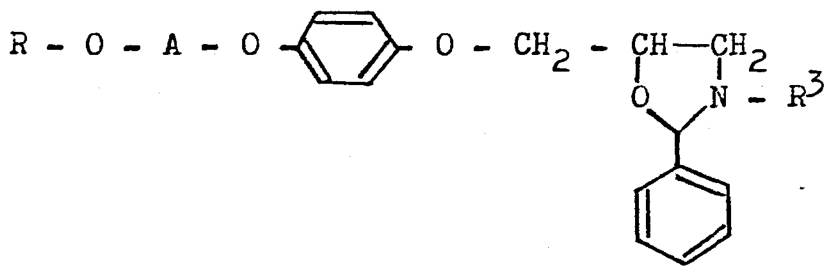 (VI)

(wherein R, $R^3$ and A are as defined above)
to hydrolysis with an acid or alkaline water.

The reaction is carried out in a suitable solvent as mentioned above by making the phenolic hydroxide group of the derivative (IV) into an alkali metal salt by treatment with potassium carbonate, sodium carbonate, potassium hydroxide, sodium hydroxide, sodium methylate, sodium hydride, sodium amide or the like and subsequently by reacting it with the oxazolidine derivative (V). It generally advances at room temperature, but may also be effected by heating up to the boiling point of the solvent used.

The hydrolysis of the compounds of formula (VI) is carried out by heating under reflux in an aqueous solution of hydrochloric acid, sulfuric acid, phosphoric acid, etc. or an aqueous solution of potassium hydroxide, sodium hydroxide, potassium carbonate, sodium carbonate, etc. Here, an alcohol such as methanol, ethanol or the like may be mixed with the solution.

The compounds of formula (I) can form an inorganic acid salt with hydrochloric acid, hydrobromic acid, sulfuric acid, phosphoric acid, etc. and an organic acid salt with oxalic acid, fumaric acid, maleic acid, tartaric acid, malic acid, o-(p-hydroxybenzoyl)benzoic acid, o-[p-(o-hydroxyphenyl)benzoyl]benzoic acid, tannic acid, etc.

The compounds of formula (I) contain asymmetric carbon atoms in their molecules, and accordingly, are present in the form of a racemate or a racemic-diastereomer as the case may be. The racemates can be resolved optically in a conventional manner. The racemic-diastereomers, if required, can be separated into one another, for example by partition crystallization, followed by resolution into the respective optical isomers.

The compounds of this invention have a blocking activity extremely selective toward $\beta_1$-receptor of the sympathetic nervous system, and are useful as a cardiac selective $\beta$-blocking drug with slight side effects as seen in other blocking drugs, such as inducement to bronchial asthma, peripheral circulation disorder, etc. Such activity is ascertained by the fact that the compounds of this invention suppress the increase in the heart beat number at a far lower dosage than restraining action of blood pressure lowering against the increase of the heart beat number and the blood pressure lowering caused by the treatment of anesthetized rats with isoproterenol which is a stimulant to $\beta$-receptor.

The compounds of this invention are clinically effective to ischemic heart diseases such as hypertension,

arrhythmia, angina pectoris or the like as a β-blocking agent. For instance, the compounds of the invention have the effect of improving the hypertension of spontaneous hypertensive rats (SHR) and the effect of suppressing the occurrence of angina pectoris due to Ouabain (when administered at a large dose, toxic to heart).

Further feature of the compounds of this invention is seen in that they exhibit, when orally administered, these effects slowly and gradually and last them long. As a consequence, when the compounds of this invention are applied to hypertension, side effects as seen in other drugs such as orthostatic dizziness due to sharp lowering in blood pressure can be avoided, and frequency of administration per day is lessened, thus alleviating a burden to the patients.

The compounds of formula (I) according to this invention can be administered orally or parenterally in the form of a pharmaceutical preparation with a suitable and conventional, pharmaceutically acceptable carrier.

The pharmaceutical preparation may take any conventional form such as tablets, capsules, granules, powders, injectable solutions, etc.

The dose of the compounds (I) usually ranges from 15 to 100 mg per day for oral administration in single dose, but can vary depending on the age, body weight and/or severity of the disease conditions to be treated as well as the response to the medication.

The invention will be hereinafter described more

specifically by way of examples, but should not be construed as being limited to them.

Example 1

To a solution of 5.7 g of 4-(2,3-epoxypropoxy)-1-[2-(2-pyridyloxy)ethoxy]benzene in 35 ml of methanol is added 5 ml of isopropylamine, and the mixture is heated under reflux for 5 hours. The reaction solution is concentrated under diminished pressure, and the resulting oily product is purified by column chromatography on silica gel to give 2.3 g of 1-{p-[2-(2-pyridyloxy)ethoxy]phenoxy}-3-isopropylamino-2-propanol as oily product. After the oily product is made into the corresponding acid maleate in isopropanol, the maleate is recrystallized from isopropanol to give 1.9 g of 1-{p-[2-(2-pyridyloxy)ethoxy]phenoxy}-3-isopropylamino-2-propanol maleate melting at 123 - 125°C.

Example 2

A mixture solution of 9.1 g of 3-bromo-1-[2-(5-bromo-2-thiazolyloxy)ethoxy]phenoxy-2-propanol, 10 ml of isopropylamine and 30 ml of acetonitrile is heated under reflux for 8 hours. After the completion of reaction, the reaction solution is concentrated under reduced pressure and the resulting oily product is purified by column chromatography on silica gel to give 3.2 g of 1-{p-[2-(5-bromo-2-thiazolyloxy)ethoxy]phenoxy}-3-isopropylamino-2-propanol as oily product. The oily product is dissolved in chloroform, and dried hydrochloric acid gas is introduced into the solution while cooling in ice to crystallize it as the corresponding hydrochloride. The crystallized product is recrystallized

from isopropanol to give 2.2 g of 1-{p-[2-(5-bromo-2-thiazolyloxy)ethoxy]phenoxy}-3-isopropylamino-2-propanol hydrochloride melting at 145 - 148°C.

Example 3

To a suspension of 0.66 g of 50 % oily sodium hydride in 25 ml of dimethylformamide is added dropwise a solution of 3.5 g of p-[2-(4-pyridyloxy)ethoxy]phenol in 15 ml of dimethylformamide while stirring under cooling in ice. After the dropwise addition, the mixture is stirred while cooling in ice for 30 minutes. Then, a solution of 8.7 g of N-tert-butyl-2-phenyl-5-paratolylsulfonyloxymethyl-1,3-oxazolidine in 25 ml of dimethylformamide is added dropwise thereto and the resulting mixture is heated while stirring at 60°C for 5 hours. After the reaction, the reaction solution is poured into water and extracted with ethyl acetate. The ethyl acetate layer is washed with water, dried over anhydrous magnesium sulfate, and the solvent is distilled off to give 2.1 g of an oily product. The oily product is dissolved in 100 ml of 2 N hydrochloric acid and the solution is refluxed under heating for one hour. After cooling, the reaction solution is made alkaline with potassium carbonate. The oily product liberated is extracted with ethyl acetate, washed with water, dried over anhydrous magnesium sulfate and subsequently, concentrated under reduced pressure. The oily product thus obtained is purified by column chromatography on silica gel to give 1.2 g of 1-{p-[2-(4-pyridyloxy)ethoxy]phenoxy}-3-tert-butylamino-2-propanol. This is made into the

corresponding acid maleate in isopropanol and the maleate is recrystallized from isopropanol to give 0.95 g of 1-{p-[2-(4-pyridyloxy)ethoxy]phenoxy}-3-tert-butylamino-2-propanol maleate melting at 133 - 135°C (decomposition).

The following compounds are also produced in accordance with the procedure of the above examples.

(1) 1-{p-[2-(4-pyridyloxy)ethoxy]phenoxy}-3-isopropylamino-2-propanol fumarate 1/2 hydrate, m.p. 158 - 159°C

(2) 1-{p-[2-(3-pyridyloxy)ethoxy]phenoxy}-3-isopropylamino-2-propanol maleate, m.p. 145 - 147°C

(3) 1-{p-[2-(2-pyrimidinyloxy)ethoxy]phenoxy}-3-isopropylamino-2-propanol hydrochloride, m.p. 119 - 120°C

(4) 1-{p-[2-(2-pyrimidinyloxy)ethoxy]phenoxy}-3-[2-(3,4-dimethoxyphenyl)ethyl]amino-2-propanol hydrochloride, m.p. 162 - 163°C

(5) 1-{p-[2-(2-pyrimidinyloxy)ethoxy]phenoxy}-3-(3-phenylpropyl)amino-2-propanol hydrochloride, m.p. 152 - 153°C

(6) 1-{p-[2- (2-thiazolyloxy)ethoxy]phenoxy}-3-isopropylamino-2-propanol maleate, m.p. 109 p 111°C

(7) 1-{p-[2-(2-pyridyloxy)ethoxy]phenoxy}-3-piperidino-2-propanol, m.p. 79 - 80°C

The invention has been fully described in the foregoing description and the examples included therein, but may be varied or modified in various ways without departing from the spirit and purview of the invention.

Claims

1. Phenoxyaminopropanol derivatives represented by the general formula:

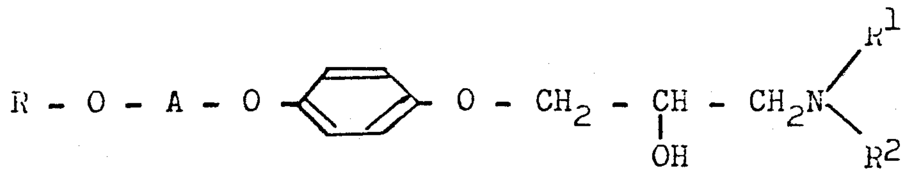

(wherein R is 2-, 3- or 4-pyridyl, 2-pyrimidyl, 2-thiazolyl or 5-bromo-2-thiazolyl; $R^1$ and $R^2$ each are hydrogen, lower alkyl, cycloalkyl or aralkyl, or form, together with the adjacent nitrogen atom, pyrrolidine or piperidine; and A is alkylene containing 2 or 3 carbon atoms ) or salts thereof.

2. A compound as claimed in claim 1, which is 1-{p-[2-(2-pyridyloxy)ethoxy]phenoxy}-3-isopropylamino-2-propanol.

3. A compound as claimed in claim 1, which is 1-{p-[2-(5-bromo-2-thiazolyloxy)ethoxy]phenoxy}-3-isopropylamino-2-propanol.

4. A compound as claimed in claim 1, which is 1-{p-[2-(4-pyridyloxy)ethoxy]phenoxy}-3-tert-butylamino-2-propanol.

5. A compound as claimed in claim 1, which is 1-{p-[2-(4-pyridyloxy)ethoxy]phenoxy}-3-isopropylamino-2-propanol.

6. A compound as claimed in claim 1, which is 1-{p-[2-(3-pyridyloxy)ethoxy]phenoxy}-3-isopropylamino-2-propanol.

7. A compound as claimed in claim 1, which is 1-{p-[2-(2-pyrimidinyloxy)ethoxy]phenoxy}-3-isopropylamino-2-propanol.

8. A compound as claimed in claim 1, which is 1-{p-[2-(2-pyrimidinyloxy)ethoxy]phenoxy}-3-[2-(3,4-dimethoxyphenyl)ethyl]amino-2-propanol.

9. A compound as claimed in claim 1, which is 1-{p-[2-(2-pyrimidinyloxy)ethoxy]phenoxy}-3-(3-phenylpropyl)-amino-2-propanol.

10. A compound as claimed in claim 1, which is 1-{p-[2-(2-thiazolyloxy)ethoxy]phenoxy}-3-isopropylamino-2-propanol.

11. A compound as claimed in claim 1, which is 1-{p-[2-(2-pyridyloxy)ethoxy]phenoxy}-3-piperidino-2-propanol.

12. A method of producing phenoxyaminopropanol derivatives represented by the general formula:

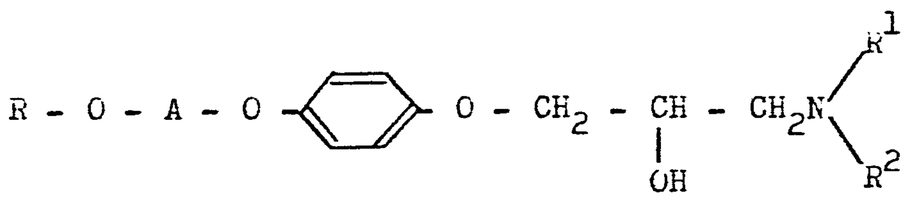

(I)

(wherein R is 2-, 3- or 4-pyridyl, 2-pyrimidyl, 2-thiazolyl or 5-bromo-2-thiazolyl; $R^1$ and $R^2$ each are hydrogen, lower alkyl, cycloalkyl or aralkyl, or form, together with the adjacent nitrogen atom, a pyrrolidine or piperidine; and A is alkylene containing 2 or 3 carbon atoms) or salts thereof, which method comprises:

(1) reacting a compound of the general formula:

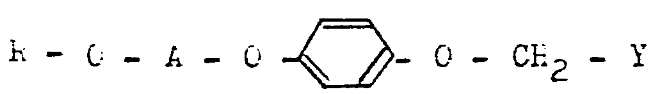

(II)

(wherein Y is 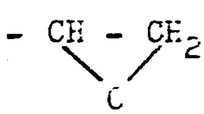 or $- CH(OH) - CH_2 - Hal$ ( Hal

represents halogen); A and R are as defined above)
with an amine of the general formula:

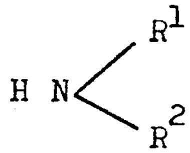 (III)

(wherein $R^1$ and $R^2$ are as defined above)
or (2) reacting a phenol derivative of the general formula:

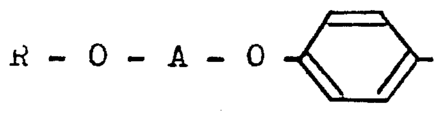 (IV)

(wherein A and R are as defined above) with an oxazolidine derivative of the general formula:

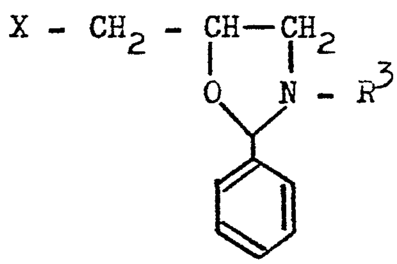 (V)

(wherein X is a reactive derivative of alcohol and $R^3$ is alkyl or aralkyl)
and hydrolyzing the resulting compound of the general formula :

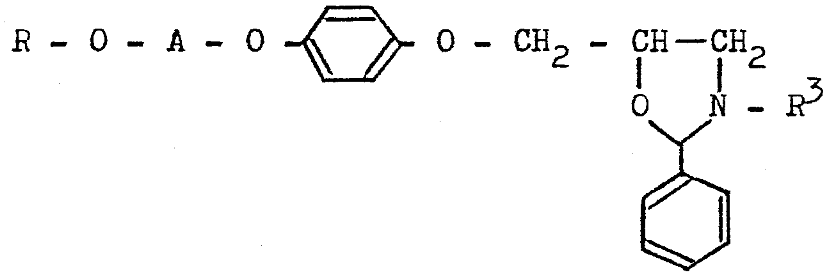 (VI)

(wherein R, $R^3$ and A are as defined above).

13. A pharmaceutical composition comprising a therapeutically effective amount of a compound as set forth in claim 1 and a pharmaceutically acceptable carrier.

# INTERNATIONAL SEARCH REPORT

International Application No. PCT/JP83/00398

**I. CLASSIFICATION OF SUBJECT MATTER** (if several classification symbols apply, indicate all)[3]

According to International Patent Classification (IPC) or to both National Classification and IPC

Int.Cl.$^3$ C07D 213/64, C07D 213/65, C07D 213/68, C07D 239/34, C07D 277/34, A61K 31/425, A61K 31/44, A61K 31/445, A61K 31/505

**II. FIELDS SEARCHED**

Minimum Documentation Searched[4]

| Classification System | Classification Symbols |
|---|---|
| I P C | C07D 213/64, C07D 213/65, C07D 213/68, C07D 239/34, C07D 277/34, A61K 31/425, A61K 31/44, A61K 31/445, A61K 31/505 |

Documentation Searched other than Minimum Documentation to the Extent that such Documents are Included in the Fields Searched[5]

**III. DOCUMENTS CONSIDERED TO BE RELEVANT**[14]

| Category* | Citation of Document,[16] with indication, where appropriate, of the relevant passages[17] | Relevant to Claim No.[18] |
|---|---|---|
| | | |

* Special categories of cited documents:[15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search[2] | Date of Mailing of this International Search Report[2] |
|---|---|
| February 6, 1984 (06. 02. 84) | February 13, 1984 (13. 02. 84) |
| International Searching Authority[1] | Signature of Authorized Officer[20] |
| Japanese Patent Office | |